# EUROPEAN PATENT APPLICATION

(11) **EP 4 276 454 A1**
(43) Date of publication of application: **15.11.2023**
(21) Application number: 22460072.6
(22) Date of filing: 15.12.2022
(51) Int. Cl.: G01N 27/403, G01N 33/38

(54) **METHOD OF PRODUCTION AND APPLICATION OF FIXED ELECTROCHEMICAL SENSORS IN CONCRETE, PARTICULARLY IN EXISTING REINFORCED CONCRETE STRUCTURES**

(30) Priority: 16.03.2022 PL 44064822
(71) Applicant: Politechnika Slaska, 44-100 Gliwice (PL)
(72) Inventor: Jasniok, Tomasz, 43-190 Mikolów (PL)

(57) **Abstract**

The method of production and application of fixed electrochemical sensors in concrete in existing reinforced concrete structures **characterized in that** the arch-shaped mesh 1 with radius r and pits 4 staggered in a few layers and one longitudinal pit 5 is prepared, wherein graphite paste 9 is placed in the pit 5 and chloride oxide paste 7 or manganese oxide paste 8 is placed in the pit 4 to form a convex meniscus, the mesh with pits filled with paste is mechanically pressed against the opening wall of opening 2 with radius r in concrete in reinforced concrete member to be hardened, then the opening is filled with resin 10, wires with connection plates inside the pits are connected to the voltage meter 11 and difference in potential between the pits, which is calibrated against the value of chloride concentration or pH, is measured.

## Description

The present invention provides a method of production and application of fixed electrochemical sensors in concrete, particularly in existing reinforced concrete structures.

Reinforced concrete structures should meet the assumed requirements for serviceability, bearing capacity and stability through their residual life without excessive maintenance costs. Unfavourable changes, which gradually deteriorate performance characteristics, are caused by environmental impact. Particularly significant are factors specified in *PN-EN 206+A2:2021-08 Concrete* - *Requirements, properties, production and compliance,* which cause destruction of both concrete and reinforcing steel. Particular attention is made to factors inducing destruction of steel reinforcement, which include the presence of chloride ions in concrete and/or reduced pH of solution in concrete pores. The exact value of chloride concentration in concrete and pH values are the substantial information for corrosion diagnostics of reinforced concrete structures.

Measurements of ion, including chloride ions, concentrations and pH values taken in electrochemical laboratories employ the potentiometric method with ion-selective membrane electrodes and conventional reference electrodes (cf. *K. Pigoń, Z. Ruziewicz: Chemia fizyczna 1. Podstawy fenomenologiczne, Wydawnictwo Naukowe PWN, Warszawa, 2007*). The ion-selective membrane plays a fundamental role in the process of formation of electrode potential. The membrane (and its composition) is chosen so as only the selected analysed ions - analyte ions, may move inside it. One side of the membrane is in contact with solution with constant concentration of analyte ions, and the other side with the analysed solution. Diffusion of analyte ions from membrane to the sample or in a reverse direction creates a difference in potentials at both sides of the membrane. A change in potential is determined with millivoltmeter with regard to the conventional reference electrode.

The above measurement method for concrete requires a sample of construction material taken by grinding layer by layer, and then preparation of solution as a mixture of crushed concrete and distilled water - cf. *A. Zybura, M. Jaśniok, and T. Jaśniok, Diagnostyka konstrukcji żelbetowych. T. 2. Badania korozji zbrojenia i w* 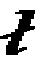 *aściwości ochronnych betonu. Warszawa: Wydawnictwo Naukowe PWN, 2011.* In such a prepared mixture chloride content is measured with the ion-selective electrode, and its pH is measured with the pH electrode. The above method is a non-destructive method, which does not allow for repeating measurements at the same point, thus it may not be the base for monitoring changes in the aggressive environment. An attempt to place electrodes in concrete to perform continuous measurements may result in failure and a lack of reliable results.

The solution for monitoring aggression level of the environment in new (already built) structures is to use Ag/AgCl electrode as an electrochemical sensor for measuring chloride content (cf. *M. Jin, L. Jiang, D. Tao, and S. Bai: Characterization of Ag*/*AgCl electrode manufactured by immersion in sodium hypochloride acid for monitoring chloride content in concrete,* Constr. Build. Mater., vol. 122, no. Supplement C, pp. 310-319, 2016 and M. A. Climent-Llorca, E. Viqueira-Pérez, and M. M. López-Atalaya: Embeddable Ag/AgCl sensors for in-situ monitoring chloride contents in concrete, Cem. Concr. Res., vol. 26, no. 8, pp. 1157-1161, 1996*).* It uses the electrode property of being reversible with respect to Cl⁻ ions, which means its potential depends on concentration of theses ions. For pH, potentiometric MMO (metal-metal-oxygen) sensors are used, whose potential depends on pH of solution, into which they are immersed. The most commonly used is iridium oxide Ir/IrO₂ (cf. S. Muralidharan et al. "Electrochemical studies on the performance characteristics of solid metal-metal oxide reference sensor for concrete environments", Sensors and Actuators, B: Chemical, vol. 113, no. 1, pp. 187-193, 2006 and G. S. Duffó, S. B. Farina, and C. M. Giordano, "Embeddable reference electrodes for corrosion monitoring of reinforced concrete structures ", Materials and Corrosion, vol. 61, no. 6, pp. 480-489, 2010), silver oxide Ag/Ag₂O (J.M. Gandiá-Romero et al.: Potentiometric thick-film sensors for measuring the pH of concrete, Cement and Concrete Composites, 2016. vol. 68. pp. 66-76*)* or manganese oxide Mn/MnO₂ (L. Telli, B. Brahimi, A. Hammouche: Study of a pH sensor with MnO2 and montmorillonite -based solidstate internal reference, Solid State Ionics. 2000, vol. 128. pp. 255-259*).*

Electrode-based sensors are prepared by anodising a bar or metal surface, however problems with their durability may occur due to oxide diffusion or loss of their activity. An alternative may be sensors produced in the microelectronic technology, known as thick-film screen printing, where chemical compounds in the form of powder which are sensitive to changes in chloride concentration and pH, are added to ink. The screen printing technique consists in transferring ink through a mesh and coating the base in a precisely specified point. The main advantage of those sensors is application of prime coat in many points of a small element, which provides the formation of meshes to determine the analysed concentrations at a depth of concrete cover *(*W.D. Huang et al.: A flexible pH sensor based on the iridium oxide sensing film, Sensors and Actuators, A: Physical, vol. 169. Elsevier, pp. 1-11, Sep. 10, 2011 and J.M. Gandiá-Romero et al.: Potentiometric thick-film sensors for measuring the pH of concrete, Cement and Concrete Composites, 2016 . vol. 68. pp. 66-76*.* Modification of inks improves durability of oxides and reduces their diffusion.

The disadvantage of the above solution is that inks may be coated on previously applied prime coat only in the form of a modified printed plate, therefore the complete product may be only used in newly-built structures, and the sensors may be placed in formwork prior to casting.

When an effort is made to apply such sensors in existing structures, it is necessary to adhere the sensor in a previously prepared deep cut, a layer of glue or binder between concrete and the sensor insulates or changes activities of measured ions at the electrode which results in false results.

Development of a new, innovative method of production and application of fixed electrochemical sensors for the existing reinforced concrete structures to eliminate the current inconveniences known from the state art, is a technical problem to be solved.

The method of production and application of the fixed electrochemical sensors in concrete in existing reinforced concrete structures **consists in the fact that** the arch-shaped mesh with radius *r* is formed with pits staggered in a few layers and one longitudinal pit, wherein graphite, chloride oxide or manganese oxide paste is put into the pits to form a convex meniscus, the mesh with pits filled with the paste is mechanically pressed against the opening wall with radius *r* in concrete of a reinforced concrete member to be hardened, then the opening is filled with resin, wires with connection plates are connected to voltmeters and difference in potential between the pits, which is calibrated against the value of chloride concentration or pH, is measured.

Preferably, in the method of production and application for concrete according to the present invention, graphite paste is prepared by dissolving two parts by weight of toluene and one part by weight of ground polystyrene granulate with particle size from 1 mm to 10 mm, and by adding up to four parts by weight of powder graphite with purity level of at least 99.5% C.

Preferably, in the method of production and application for concrete according to the present invention, silver oxide paste is prepared by dissolving two parts by weight of toluene and one part by weight of crushed polystyrene granulate with particle size from 1 mm to 10 mm, and by adding up to four parts by weight of silver oxide.

Preferably, in the method of production and application for concrete according to the present invention, manganese oxide paste is prepared by dissolving two parts by weight of toluene and one part by weight of ground polystyrene granulate with particle size from 1 mm to 10 mm, and by adding up to four parts by weight of manganese (IV) oxide.

The advantage of this solution is the possibility or monitoring continuously changes in chloride ions concentration or a drop in concrete pH at the depth of concrete cover when the sensor is applied for concrete in the existing structure.

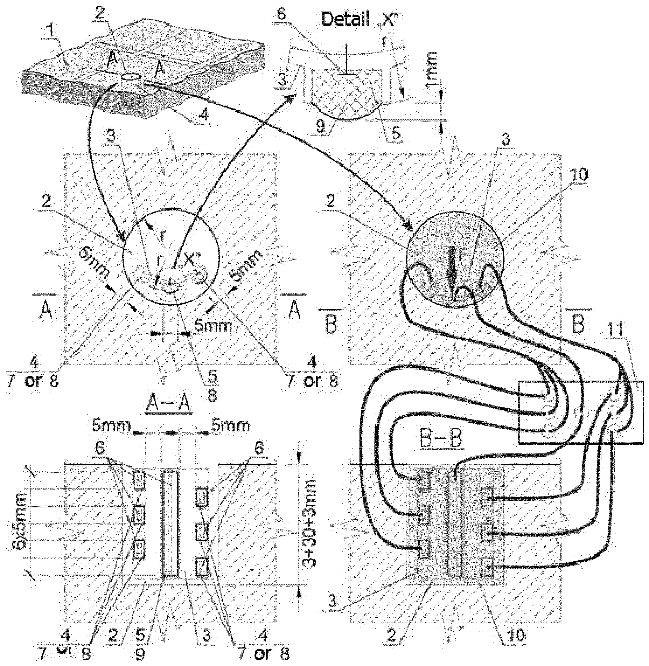

The object of the invention in the embodiment is illustrated in the drawing as a diagram of the method of production and application of the sensor in the slab element of a reinforced concrete structure.

### Embodiment

When starting to perform and apply the sensor for chloride ions concentration or pH sensor in the slab-type reinforced concrete member **1**, an opening **2** is performed with radius **r** and a height of 36 mm (the height of a line of pits and additionally 3 mm from both top and bottom), to which the plastic arched mesh **3** with radius **r** is introduced. The mesh consists of 6 square pits **4** with internal dimensions of 5×5 mm and a depth of 2 mm, and one longitudinal pit **5** with internal dimensions of 5×30 mm and a depth of 2 mm. The pit **5** is placed in a centre of the mesh to ensure the same distance to each pit **4** equal to 5 mm. However, the pits **4** are staggered in 6 rows on both sides of the pit **5**, so as they are within a distance of 5 mm from one another. Arrangement of the pits determines dimensions of the mesh **3**, whose height is 32 mm and arc width **r** measured from outer edges of the pits is 27 mm. Inside the pits, there are connection plates **6** made of sliver sample minimum 925, to which the wires are connected. The pits **4** are filled with the paste **7** prepared by dissolving two parts by weight of toluene, one part by weight of crushed polystyrene granulate with particle size not greater than 1 mm, and by adding four parts by weight of silver chloride in case of production of a sensor for monitoring changes in chloride concentration, or the paste **8** prepared by dissolving two parts by weight of toluene, one part by weight of crushed polystyrene granulate with particle size not greater than 1 mm, and by adding four parts by weight of manganese (IV) oxide in case of production of a sensor for monitoring pH. The pit **5** is filled with the paste **9** prepared by dissolving two parts by weight of toluene and one part by weight of crushed polystyrene granulate with particle size not greater than 1 mm, and by adding four parts by weight of powder graphite with purity level of at least 99.5% C. Paste in all the pits must form a convex meniscus with a height of 1 mm. Then, the mesh with pits filled with relevant pastes is pressed down mechanically with force **F** to the opening wall **2** and the force is maintained for 24 hours under dry air conditions until the coating is hardened due to toluene evaporation. After that time, the opening **2** is filled with epoxy resin **10** to stabilize the adhered mesh, and the wires are connected to the voltage meter **11** with high internal resistance, and a difference in potential between the electrode **9** and the other electrodes **7** or **8** is measured.

## Claims

1. A method of production and application of fixed electrochemical sensors in concrete in existing reinforced concrete structures **characterized in that** an arch-shaped mesh **3** with radius **r** and pits **4** staggered in a few layers and one longitudinal pit **5** is prepared, wherein graphite paste **9** is placed in the pit **5**, and chloride oxide paste **7** or manganese oxide paste **8** is placed in the pit **4** to form a convex meniscus, the mesh with the filled pits is mechanically pressed against the opening wall **2** with radius **r** in concrete in reinforced concrete member to be hardened, then the opening is filled with resin **9**, wires with connection plates **6** are connected to a voltmeter **11** and difference in potential between the pits, which is calibrated against the value of chloride concentration or pH, is measured.

2. A method of production and application according to claim 1 **characterized in that** graphite paste **9** is prepared by dissolving two parts by weight of toluene and one part by weight of crushed polystyrene granulate with particle size from 1 mm to 10 mm, and by adding up to four parts by weight of powder graphite with purity level of at least 99.5% C.

3. A method of production and application according to claim 1 **characterized in that** silver oxide paste **7** is prepared by dissolving two parts by weight of toluene and one part by weight of crushed polystyrene granulate with particle size from 1 mm to 10 mm, and by adding up to four parts by weight of silver oxide.

4. A method of production and application according to claim 1 **characterized in that** manganese oxide paste **8** is prepared by dissolving two parts by weight of toluene and one part by weight of crushed polystyrene granulate with particle size from 1 mm to 10 mm, and by adding up to four parts by weight of manganese (IV) oxide.
